# EUROPÄISCHE PATENTSCHRIFT

(11) **EP 4 475 909 B1**
(45) Veröffentlichungstag und Bekanntmachung des Hinweises auf die Patenterteilung: **10.12.2025**
(21) Anmeldenummer: 23734448.6
(22) Anmeldetag: 09.06.2023
(51) Int. Cl.: A61M 5/145

(54) **DOSIERSYSTEM**
DOSING SYSTEM
SYSTÈME DE DOSAGE

(30) Priorität: 01.07.2022 DE 102022116513
(43) Veröffentlichungstag der Anmeldung: 18.12.2024
(73) Patentinhaber: LENUS Infusionstechnik GmbH & Co.KG, 24118 Kiel (DE)
(72) Erfinder: KÖLLN, Rasmus, 24232 Schönkirchen (DE); MARTSCHENKO, Sven, 24113 Kiel (DE); STUMPP, Uwe, 78665 Frittlingen (DE)
(74) Vertreter: Lobemeier, Martin Landolf
(86) Internationale Anmeldenummer: PCT/DE2023/100436
(87) Internationale Veröffentlichungsnummer: WO 2024/002410

(56) Entgegenhaltungen:
- EP-A1- 1 725 281
- CA-A1- 2 130 727
- DE-A1- 102020 121 665
- US-B1- 6 645 177

## Beschreibung

Die Erfindung betrifft ein Dosiersystem, das insbesondere als mobile Spritzenpumpe Verwendung finden kann. Die Erfindung betrifft speziell ein Dosiersystem mit einer Spritze, die einen eine Düse aufweisenden Spritzenkörper und einen im Spritzenkörper verschieblich angeordneten, einen Kolbenkopf aufweisenden Spritzenkolben aufweist, und einer Spritzenpumpe, die einen Pumpenkörper, eine am Pumpenkörper angeordnete Spritzenhalterung zur Lagerung der Spritze an der Spritzenpumpe und einen eine Gewindestange antreibenden Motor zum Antrieb des Spritzenkolbens der Spritze aufweist, wobei der Spritzenkolben eine zum Aufnehmen wenigstens eines Teilabschnitts der Gewindestange eingerichtete erste Aufnahme und die Gewindestange einen mit der Gewindestange schraubenden und auf den Spritzenkolben wirkenden Schlitten aufweist.

Ein derartiges Dosiersystem ist insbesondere aus der EP 1 219 312 A2 und der DE 10 2020 121 665 A1 bekannt.

Die Besonderheit dieser Dosiersysteme besteht darin, dass der Kolben der Spritze so ausgeführt ist, dass die Gewindestange vom Kolben aufgenommen wird. Im Übrigen ist das Funktionsprinzip für die Förderung der Flüssigkeit identisch zu normalen Perfusoren. Die Spindel wird um die eigene Achse gedreht, wodurch der Schlitten, der drehfest auf der Spindel sitzt, den Kolben in Richtung der Spritzendüse treibt und damit die Flüssigkeit aus der Spritze drückt. Um sicherzustellen, dass sich der Kolben in der Spritze bewegt und nicht die gesamte Spritze vom Kolben aus dem Pumpenkörper herausgedrückt wird, ist an der Spritzenpumpe üblicherweise ein Bajonett-Verschluss vorgesehen, mit dem der Spritzenkörper mit dem Pumpenkörper verspannt wird. Damit ist sichergestellt, dass die Spritze selbst nicht bewegt wird, sondern der Kolben innerhalb der Spritze.

Ein Nachteil dieser Dosiersysteme besteht darin, dass diese ausschließlich zum Entleeren, nicht aber zum Befüllen der Spritzen eingerichtet sind. So sind für das Befüllen von Spritzen, die im Rahmen der bekannten Dosiersysteme Verwendung finden sollen, gesonderte Füllhilfen bekannt, mit denen die Spritzen befüllt werden, wobei die befüllte Spritze anschließend in die Spritzenpumpe zur Verwendung im Dosiersystem eingesetzt werden kann.

Weitere Dosiersysteme sind aus der EP 1 725 281 A1, der CA 2 130 727 A1 und der US 6 645 177 B bekannt.

Aufgabe der Erfindung ist es daher, ein Dosiersystem zu schaffen, mit dem ein automatisiertes Entleeren und Befüllen der Spritze mit einer Spritzenpumpe möglich ist.

Diese Aufgabe wird erfindungsgemäß durch das Dosiersystem mit den Merkmalen von Anspruch 1 gelöst. Die Unteransprüche geben vorteilhafte Ausgestaltungen der Erfindung wieder.

Grundgedanke der Erfindung ist es, den mit der Gewindestange schraubenden Schlitten, der üblicherweise vollständig drehfest gelagert ist, in einem vorbestimmten Winkelmaß pendelnd zu lagern, damit dieser über eine vorbestimmte Strecke mit der Gewindestange mitdrehen kann. Diese begrenzte Drehbewegung, die eine Förderung von von der Spritze aufgenommenem Fluid nicht bewirken kann, wird zur Ausbildung eines lösbaren Formschlusses zwischen dem Schlitten und dem Spritzenkolben genutzt, wobei der für den Schlitten in der jeweiligen Drehrichtung vorgesehene Anschlag bei weiterem Antrieb der Gewindestange in derselben Drehrichtung für eine drehfeste Lagerung des Schlittens sorgt. Damit aber ist es in nur einer Vorrichtung ermöglicht, dass der Schlitten den Spritzenkolben automatisiert in den Pumpenkörper ziehen und die Spritze mit einem Fluid, insbesondere einem Medikament befüllt werden kann.

Bei entgegengesetzter Drehrichtung zum Entleeren der Spritze wird der Formschluss aufgehoben, sodass der Schlitten - der wiederum an einem in der Gegenrichtung vorgesehenen Anschlag drehfest gelagert ist - den Spritzenkolben aus dem Pumpenkörper in die Spritze schiebt und die Spritze insgesamt ohne weiteres im entleerten Zustand entnommen werden kann.

Der Vorteil der vorliegenden Erfindung liegt somit darin, dass eine mit einer Spritzenpumpe verwendete Spritze in entleertem Zustand in die Spritzenpumpe eingesetzt und mit dieser befüllt sowie entleert werden kann, ohne dass es einer weiteren für das Befüllen der Spritze erforderlichen Vorrichtung bedarf. Das erfindungsgemäße Dosiersystem ist damit für die einfache Handhabung durch auf die Versorgung mit Medikamenten mittels der an sich bekannten Dosiersysteme besonders geeignet.

Erfindungsgemäß wird also ein Dosiersystem mit einer Spritze, die einen eine Düse aufweisenden Spritzenkörper und einen im Spritzenkörper verschieblich angeordneten, einen Kolbenkopf aufweisenden Spritzenkolben aufweist, und einer Spritzenpumpe, die einen Pumpenkörper, eine am Pumpenkörper angeordnete Spritzenhalterung zur Lagerung der Spritze an der Spritzenpumpe und einen eine Gewindestange antreibenden Motor zum Antrieb des Spritzenkolbens der Spritze aufweist, vorgeschlagen, wobei der Spritzenkolben eine zum Aufnehmen wenigstens eines Teilabschnitts der Gewindestange eingerichtete erste Aufnahme und die Gewindestange einen mit der Gewindestange schraubenden und auf den Spritzenkolben wirkenden Schlitten aufweist. Der Kolbenkopf ist insbesondere als am Spritzenkolben endständiger Flansch nach Art einer Daumenauflage ausgebildet. Das erfindungsgemäße Dosiersystem ist weiter derart ausgebildet, dass der Schlitten in einem vorbestimmten von jeweils einem Anschlag begrenzten Winkelmaß mit der Gewindestange mitdrehend und bei Anliegen des Schlittens an dem in der von der Gewindestange ausgeübten Drehrichtung angeordneten Anschlag drehfest eingerichtet ist. Zusätzlich weisen der Spritzenkolben und der Schlitten jeweils ein Verbindungsmittel zur Ausbildung eines den Spritzenkolben und Schlitten miteinander verbindenden Formschlusses auf, sodass der Spritzenkolben und der Schlitten bei Anliegen des Schlittens an dem einen Anschlag den Formschluss von Spritzenkolben und Schlitten ausbildend miteinander verbunden und bei Anliegen des Schlittens an dem anderen Anschlag voneinander getrennt sind.

Aufgrund dieser Ausgestaltung ist es möglich, dass der Schlitten eine begrenzte Drehbewegung ausführt, die für das Ausbilden und Trennen eines Formschlusses von Schlitten und Spritzenkolben genutzt wird, wobei die von der Spritzenpumpe gebildeten, die Drehbewegung begrenzenden Anschläge für eine drehfeste Lagerung des Schlittens sorgen.

Bevorzugt weist der Spritzenkolben eine zur Aufnahme des am Schlitten ausgebildeten Verbindungsmittels eingerichtete Ausnehmung auf. Hierbei kann es sich bespielsweise um eine am Spritzenkolben, insbesondere am Kolbenkopf vorgesehene Öffnung handeln, in die das am Schlitten vorgesehene Verbindungsmittel eintreten und mittels Drehung gesichert werden kann. Besonders bevorzugt ist das am Schlitten ausgebildete Verbindungsmittel ein Rastmittel. Speziell ist das am Schlitten ausgebildete Verbindungsmittel ein Haken. Jedenfalls sind die Verbindungsmittel so ausgebildet, dass die gegenseitige Verschiebung von Schlitten und Spritzenkolben in axialer Richtung des Spritzenkolbens bei bestehendem Formschluss verhindert ist. Vielmehr wird eine Bewegung des Schlittens bei bestehendem Formschluss unmittelbar auf den Spritzenkolben übertragen, sodass insbesondere ein Befüllen der Spritze erfolgen kann. Für das Entleeren der Spritze hingegen ist ein Formschluss nicht erforderlich, da der Spritzenkolben für das Entleeren lediglich vom Schlitten geschoben werden muss.

Gemäß einer höchst bevorzugten Ausgestaltung weist das am Schlitten angeordnete Verbindungsmittel auf seiner dem Spritzenkolben zugewandten Seite eine mit dem am Spritzenkolben angeordneten Verbindungsmittel kooperierende, zur Positionierung des Schlittens in eine zur Ausbildung des Formschlusses vorbereitende Position eingerichtete Leitstruktur auf. Dabei kann es sich um eine Schräge handeln, die beispielsweise dazu führt, dass ein am Schlitten als Haken ausgebildetes Verbindungsmittel so positioniert wird, dass der Haken in eine am Spritzenkolben vorgesehene Öffnung eintreten kann.

Schließlich beträgt das Winkelmaß, in dem der Schlitten eine pendelnde Bewegung ausüben kann, bevorzugt ≤ 25° und besonders bevorzugt ≤ 20°. Die Pendelbewegung ist jedenfalls derart dimensioniert, dass ein durch die Pendelbewegung bewirkter Formschluss bei Umkehr der Drehrichtung der Gewindestange auch vollständig aufgehoben und die Spritze sicher aus der Pumpe entfernt werden kann.

Die Erfindung wird im Folgenden anhand eines in den beigefügten Zeichnungen dargestellten, besonders bevorzugt ausgestalteten Ausführungsbeispiels näher erläutert. Es zeigen:
- Fig. 1: eine teilweise geschnittene Ansicht des aus Spritze und Spritzenpumpe bestehenden Dosiersystems in einem voneinander getrennten Zustand (A) und in einem miteinander verbundenen Zustand (B);
- Fig. 2: den Vorgang der Spritzenbefüllung mittels des bevorzugt ausgestalteten Dosiersystems zu Beginn des Füllvorgangs in einem Querschnitt auf Höhe der Verbindung zwischen Schlitten und Spritzenkolben (A) und in einem Längsschnitt (B) sowie nach Beendigung der Befüllung in einem Querschnitt auf Höhe der Verbindung zwischen Schlitten und Spritzenkolben (C) und in einem Längsschnitt (D);
- Fig. 3: die Pendelbewegung des Schlittens aus einer Position, in der der Schlitten nicht im Eingriff mit dem Spritzenkolben ist (A), in eine den Spritzenkolben in der Spritzenpumpe verriegelnden Position (B);
- Fig. 4: den Vorgang der Spritzenentleerung mittels des bevorzugt ausgestalteten Dosiersystems vor der Entleerung in einem Querschnitt auf Höhe der Verbindung zwischen Schlitten und Spritzenkolben (A) und in einem Längsschnitt (B) sowie gegen Ende der Entleerung in einem Querschnitt auf Höhe der Verbindung zwischen Schlitten und Spritzenkolben (C) und in einem Längsschnitt (D); und
- Fig. 5: die Pendelbewegung des Schlittens von der den Spritzenkolben in der Spritzenpumpe verriegelnden Position (A) in die den Spritzenkolben freigebenden Position (B).

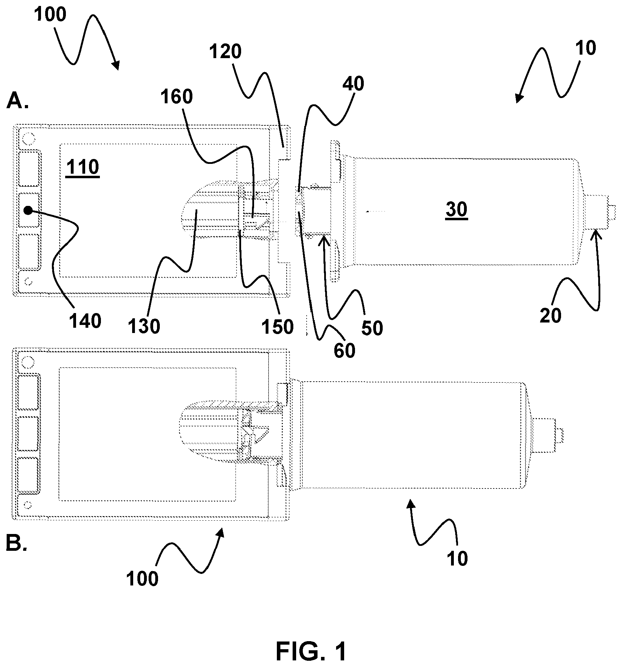

Fig. 1 zeigt eine teilweise geschnittene Ansicht des aus Spritze und Spritzenpumpe bestehenden Dosiersystems in getrenntem Zustand (A) und in miteinander verbundenem Zustand (B). Insbesondere zeigt Fig. 1 ein besonders bevorzugt ausgestaltetes Dosiersystem, das aus einer Spritze 10 und einer Spritzenpumpe 100 besteht.

Die Spritze 10 weist einen mit einer Düse 20 ausgebildeten Spritzenkörper 30 auf, in dem ein einen Kolbenkopf 40 aufweisender Spritzenkolben 50 verschieblich angeordnet ist.

Die Spritzenpumpe 100 weist einen Pumpenkörper 110 mit einer am Pumpenkörper 110 angeordneten Spritzenhalterung 120 auf, die zur Lagerung der Spritze 10 an der Spritzenpumpe 100 eingerichtet ist. Des Weiteren weist die Spritzenpumpe 100 eine von einem Motor 140 angetriebene Gewindestange 130 auf, die zum Antrieb des Spritzenkolbens 50 der Spritze 10 eingerichtet ist. Hierfür ist der Spritzenkolben 50 mit einer zum Aufnehmen wenigstens eines Teilabschnitts der Gewindestange 130 eingerichteten Aufnahme und die Gewindestange 130 mit einem mit der Gewindestange 130 schraubenden und auf den Spritzenkolben 50 wirkenden Schlitten 150 eingerichtet.

Entgegen den bekannten Systemen, bei denen der Schlitten zur ausschließlichen Bewegung in - auf die Längsachse der Gewindestange 130 bezugnehmend - axialer Richtung eingerichtet ist, ist der Schlitten 150 in einem vorbestimmten von jeweils einem Anschlag begrenzten Winkelmaß mit der Gewindestange 130 mitdrehend eingerichtet und nur bei Anliegen des Schlittens 150 an dem in der von der Gewindestange 130 ausgeübten Drehrichtung angeordneten Anschlag drehfest.

Mit Hilfe der am Spritzenkolben 50 und am Schlitten 150 vorgesehenen Verbindungsmittel 60, 160 zur Ausbildung eines den Spritzenkolben 50 und Schlitten 150 miteinander verbindenden Formschlusses wird so eine erste Position eingenommen, in der der Spritzenkolben 50 und der Schlitten 150 bei Anliegen des Schlittens 150 an dem einen Anschlag den Formschluss von Spritzenkolben 50 und Schlitten 150 ausbildend miteinander verbunden sind und eine zweite Position ermöglicht, in der der Spritzenkolben 50 und der Schlitten 150 bei Anliegen des Schlittens 150 an dem anderen Anschlag voneinander getrennt sind.

Diese Ausgestaltung ermöglicht - wie die folgenden Abbildungen zeigen - sowohl das automatisierte Befüllen der Spritze 10 durch Einziehen des Spritzenkolbens 50 in den Pumpenkörper 110 mittels des Schlittens 150 wie auch das automatisierte Entleeren der Spritze 10 mittels des Schlittens 150 sowie die ungehinderte Entnahme der Spritze 10 aus dem Pumpenkörper 110.

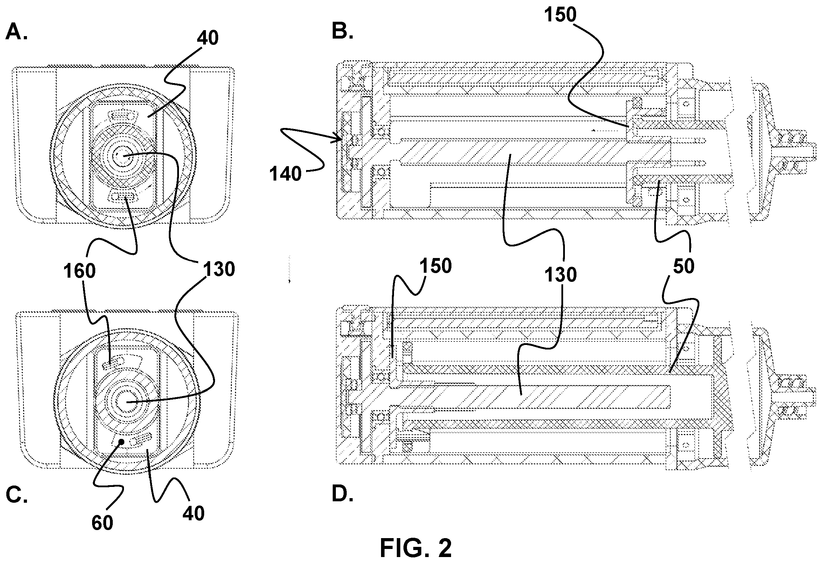

Zunächst zeigt Fig. 2 den Vorgang der Spritzenbefüllung mittels des bevorzugt ausgestalteten Dosiersystems zu Beginn des Füllvorgangs in einem Querschnitt auf Höhe der Verbindung zwischen Schlitten 150 und Spritzenkolben 50 (Fig. 2A) und in einem Längsschnitt (Fig. 2B) sowie nach Beendigung der Befüllung in einem Querschnitt auf Höhe der Verbindung zwischen Schlitten 150 und Spritzenkolben 50 (Fig. 2C) und in einem Längsschnitt (Fig. 2D).

Die Spritze 10 wird in die Spritzenpumpe 100 eingesetzt und mittels speziell als Bajonettverschluss ausgebildeten Spritzenhalterung 120 am Pumpenkörper 110 drehfest und positionsfest fixiert. Der Kolbenkopf 40 des Spritzenkolbens weist als Verbindungsmittel 60 eine Ausnehmung 60 zur Aufnahme des am Schlitten 150 ausgebildeten Verbindungsmittels 160 auf. Dabei weist das am Schlitten 150 angeordnete Verbindungsmittel 160 auf seiner dem Spritzenkolben 50 zugewandten Seite eine mit dem am Spritzenkolben 50 angeordneten Verbindungsmittel 60 kooperierende, zur Positionierung des Schlittens 150 in eine zur Ausbildung des Formschlusses vorbereitende Position eingerichtete Leitstruktur auf. Diese Leitstruktur ist insbesondere als Schräge ausgebildet, sodass - sollte sich der Schlitten 150 nicht in einer zur Ausbildung des Formschlusses geeigneten Ausgangsposition befinden - der Schlitten 150 durch Einwirken des Spritzenkolbens 50 auf das Verbindungsmittel 160 in die geeignete (Ausgangs-) Position gedreht werden kann, in der das Verbindungsmittel 160 in die am Kolbenkopf 40 gebildete Ausnehmung 60 eintreten kann.

Wird nun der Schlitten - wie in Fig. 2A angedeutet - im vorliegenden Fall gegen den Uhrzeigersinn gedreht, wird mittels Formschluss zwischen den Verbindungsmitteln 60, 160 eine Verbindung von Schlitten 150 und Spritzenkolben 50 geschaffen, die es erlaubt, dass der Spritzenkolben 50 in den Spritzenkörper 110 gezogen wird.

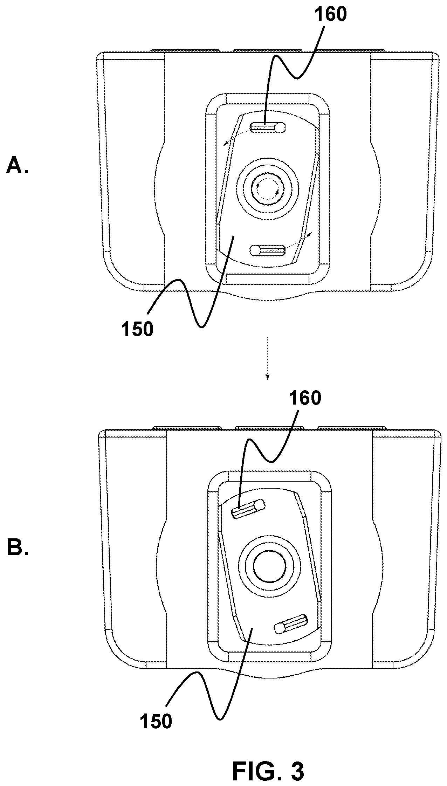

Die axiale Bewegung des Schlittens 150 entlang der Gewindestange 130 wird - wie Fig. 3 zeigt - dadurch gewährleistet, dass sich der das Verbindungsmittel 160 aufweisende Schlitten 150 in einem vorbestimmten Winkelmaß bei Antrieb der Gewindestange 130 mit dieser mitdrehen kann, bis der Schlitten 150 an einen vom Pumpenkörper 110 gebildeten Anschlag anstößt und bei weiterer Drehung der Gewindestange 130 in derselben Drehrichtung drehfest gelagert ist und sich entlang der Längsachse der Gewindestange 130 bewegt. Bei dieser in axialer Richtung ausgeführten Bewegung des Schlittens 150 nimmt der Schlitten den Spritzenkolben 50 aufgrund des von Schlitten 150 und Spritzenkolben 50 bewirkten Formschlusses mit, sodass die Spritze 10 aufgezogen und damit eine Befüllung der Spritze 10 erfolgen kann.

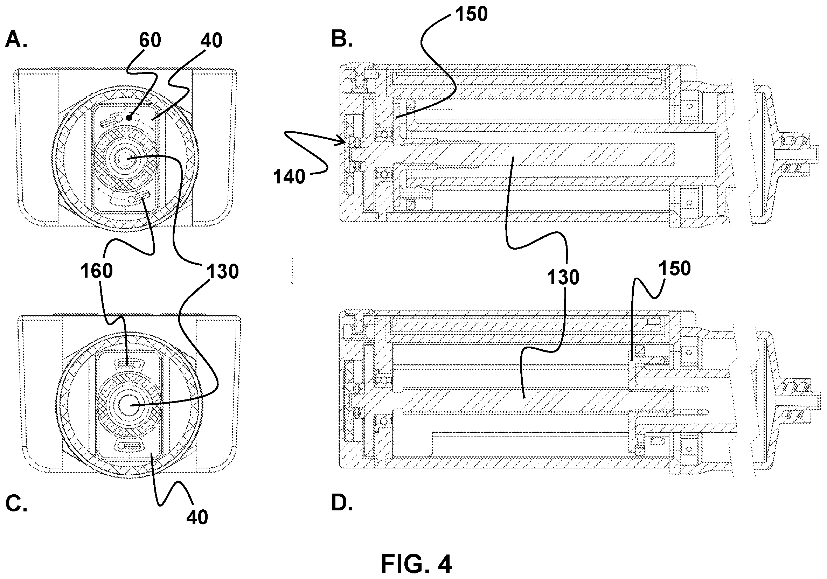

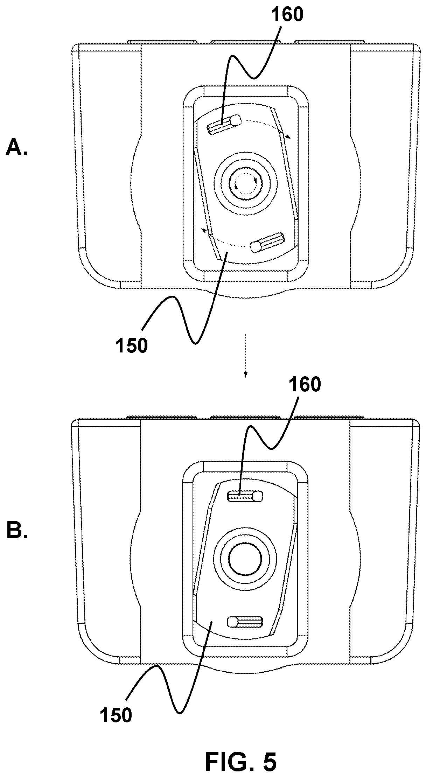

Der Vorgang der Spritzenentleerung - der in Fig. 4 und Fig. 5 dargestellt ist - erfolgt nun in umgekehrter Reihenfolge. Dabei wird die Gewindestange 130 im Verhältnis zu den vorherigen Abbildungen im Gegensinn angetrieben, sodass sich der Schlitten 150 im vorgegebenen Winkelmaß mit der Gewindestange 130 nun im Gegensinn mitdreht bis der in dieser Drehrichtung vorgesehene Anschlag erreicht ist und der Schlitten 150 drehfest gelagert ist. Der gesamte von der Pendelbewegung des Schlittens 150 überstrichene Winkelbereich beträgt im gezeigten Beispiel etwa 20°.

## Patentansprüche

1. Dosiersystem mit
- einer Spritze (10), die einen eine Düse (20) aufweisenden Spritzenkörper (30) und einen im Spritzenkörper (30) verschieblich angeordneten, einen Kolbenkopf (40) aufweisenden Spritzenkolben (50) aufweist, und
- einer Spritzenpumpe (100), die einen Pumpenkörper (110), eine am Pumpenkörper (110) angeordnete Spritzenhalterung (120) zur Lagerung der Spritze (10) an der Spritzenpumpe (100) und einen eine Gewindestange (130) antreibenden Motor (140) zum Antrieb des Spritzenkolbens (50) der Spritze (10) aufweist,
wobei der Spritzenkolben (50) eine zum Aufnehmen wenigstens eines Teilabschnitts der Gewindestange (130) eingerichtete Aufnahme und die Gewindestange (130) einen mit der Gewindestange (130) schraubenden und auf den Spritzenkolben (50) wirkenden Schlitten (150) aufweist,
**dadurch gekennzeichnet, dass**
der Schlitten (150) in einem vorbestimmten von jeweils einem Anschlag begrenzten Winkelmaß mit der Gewindestange (130) mitdrehend und bei Anliegen des Schlittens (150) an dem in der von der Gewindestange (130) ausgeübten Drehrichtung angeordneten Anschlag drehfest eingerichtet ist,
der Spritzenkolben (50) und der Schlitten (150) jeweils ein Verbindungsmittel (60, 160) zur Ausbildung eines den Spritzenkolben (50) und Schlitten (150) miteinander verbindenden Formschlusses aufweisen,
wobei der Spritzenkolben (50) und der Schlitten (150) bei Anliegen des Schlittens (150) an dem einen Anschlag den Formschluss von Spritzenkolben (50) und Schlitten (150) ausbildend miteinander verbunden und bei Anliegen des Schlittens (150) an dem anderen Anschlag voneinander getrennt sind.

2. Dosiersystem nach Anspruch 1, **dadurch gekennzeichnet, dass** der Spritzenkolben (50) eine zur Aufnahme des am Schlitten (150) ausgebildeten Verbindungsmittels (160) eingerichtete Ausnehmung (60) aufweist.

3. Dosiersystem nach einem der vorhergehenden Ansprüche, **dadurch gekennzeichnet, dass** das am Schlitten (150) ausgebildete Verbindungsmittel (160) ein Rastmittel ist.

4. Dosiersystem nach einem der vorhergehenden Ansprüche, **dadurch gekennzeichnet, dass** das am Schlitten (150) ausgebildete Verbindungsmittel (160) ein Haken ist.

5. Dosiersystem nach einem der vorhergehenden Ansprüche, **dadurch gekennzeichnet, dass** das am Schlitten (150) angeordnete Verbindungsmittel (160) auf seiner dem Spritzenkolben (50) zugewandten Seite eine mit dem am Spritzenkolben (50) angeordneten Verbindungsmittel (60) kooperierende, zur Positionierung des Schlittens (150) in eine zur Ausbildung des Formschlusses vorbereitende Position eingerichtete Leitstruktur aufweist.

6. Dosiersystem nach einem der vorhergehenden Ansprüche, **dadurch gekennzeichnet, dass** das Winkelmaß ≤ 25° ist.

## Claims

1. Dosing system having
- a syringe (10) which has a syringe body (30) having a nozzle (20) and a syringe plunger (50) which is displaceably arranged in the syringe body (30) and has a plunger head (40), and
- a syringe pump (100), which has a pump body (110), a syringe holder (120) arranged on the pump body (110) for mounting the syringe (10) on the syringe pump (100) and a motor (140) driving a threaded rod (130) for driving the syringe plunger (50) of the syringe (10),
wherein the syringe plunger (50) has a receptacle arranged to receive at least a partial section of the threaded rod (130) and the threaded rod (130) has a carriage (150) screwing with the threaded rod (130) and acting on the syringe plunger (50),
**characterized in that**
the carriage (150) rotates with the threaded rod (130) at a predetermined angular dimension limited by a respective stop and is fixed against rotation when the carriages (150) abuts against the stop arranged in the direction of rotation exerted by the threaded rod (130),
the syringe plunger (50) and the carriage (150) each have a connecting means (60, 160) for forming a positive fit connecting the syringe plunger (50) and the carriage (150) to one another,
wherein the syringe plunger (50) and the carriage (150) are connected to one another, forming the positive fit of syringe plunger (50) and carriage (150), when the carriage (150) is in contact with one stop and are separated from one another when the carriage (150) is in contact with the other stop.

2. Dosing system according to claim 1, **characterized in that** the syringe plunger (50) has a recess (60) designed to receive the connecting means (160) formed on the carriage (150).

3. Dosing system according to one of the preceding claims, **characterized in that** the connecting means (160) formed on the carriage (150) is a latching means.

4. Dosing system according to one of the preceding claims, **characterized in that** the connecting means (160) formed on the carriage (150) is a hook.

5. Dosing system according to one of the preceding claims, **characterized in that** the connecting means (160) arranged on the carriage (150) has, on its side facing the syringe plunger (50), a guide structure which cooperates with the connecting means (60) arranged on the syringe plunger (50) and is set up for positioning the carriage (150) in a position which prepares for the formation of the form fit.

6. Dosing system according to one of the preceding claims, **characterized in that** the angular dimension is ≤ 25°.

## Revendications

1. Système de dosage comportant
- une seringue (10) qui présente un corps de seringue (30) présentant une buse (20) et un piston de seringue (50) disposé de manière à pouvoir coulisser dans le corps de seringue (30) et présentant une tête de piston (40), et
- une pompe à seringue (100) qui présente un corps de pompe (110), un support pour seringue (120) disposé sur le corps de pompe (110) pour le montage de la seringue (10) sur la pompe à seringue (100) et un moteur (140) entraînant une tige filetée (130) pour l'entraînement du piston de seringue (50) de la seringue (10),
dans lequel le piston de seringue (50) présente un logement conçu pour la réception d'au moins une section partielle de la tige filetée (130) et la tige filetée (130) présente un chariot (150) se vissant à la tige filetée (130) et agissant sur le piston de seringue (50),
**caractérisé en ce que**
le chariot (150) est conçu de manière à tourner avec la tige filetée (130) dans une mesure angulaire prédéterminée limitée par respectivement une butée et, lorsque le chariot (150) s'appuie contre la butée disposée dans le sens de rotation exercé par la tige filetée (130), il est conçu de manière à être bloqué en rotation,
le piston de seringue (50) et le chariot (150) présentent respectivement un moyen de liaison (60, 160) pour la formation d'une liaison par complémentarité de forme reliant le piston de seringue (50) et le chariot (150) entre eux,
dans lequel le piston de seringue (50) et le chariot (150) sont reliés l'un à l'autre de manière à former la liaison par complémentarité de forme du piston de seringue (50) et du chariot (150) lorsque le chariot (150) s'appuie contre l'une des butées, et sont séparés l'un de l'autre lorsque le chariot (150) s'appuie contre l'autre butée.

2. Système de dosage selon la revendication 1, **caractérisé en ce que** le piston de seringue (50) présente un évidement (60) conçu pour la réception du moyen de liaison (160) réalisé sur le chariot (150).

3. Système de dosage selon l'une des revendications précédentes,
**caractérisé en ce que** le moyen de liaison (160) réalisé sur le chariot (150) est un moyen d'encliquetage.

4. Système de dosage selon l'une des revendications précédentes,
**caractérisé en ce que** le moyen de liaison (160) réalisé sur le chariot (150) est un crochet.

5. Système de dosage selon l'une des revendications précédentes,
**caractérisé en ce que** le moyen de liaison (160) disposé sur le chariot (150) présente, sur son côté tourné vers le piston de seringue (50), une structure de guidage coopérant avec le moyen de liaison (60) disposé sur le piston de seringue (50) et conçue pour le positionnement du chariot (150) dans une position préparatoire à la formation de la liaison par complémentarité de forme.

6. Système de dosage selon l'une des revendications précédentes,
**caractérisé en ce que** la mesure angulaire est ≤ 25°.
